# EUROPÄISCHE PATENTSCHRIFT

(11) **EP 2 726 625 B1**
(45) Veröffentlichungstag und Bekanntmachung des Hinweises auf die Patenterteilung: **29.07.2015**
(21) Anmeldenummer: 12729619.2
(22) Anmeldetag: 25.06.2012
(51) Int. Cl.: C12P 13/12, C12P 17/14

(54) **VERFAHREN ZUR FERMENTATIVEN PRODUKTION VON NATÜRLICHEM L-CYSTEIN**
METHOD FOR PRODUCTION OF NATURAL L-CYSTEINE BY FERMENTATION
PROCÉDÉ DE PRODUCTION PAR FERMENTATION DE L-CYSTÉINE NATURELLE

(30) Priorität: 30.06.2011 DE 102011078481
(43) Veröffentlichungstag der Anmeldung: 07.05.2014
(73) Patentinhaber: Wacker Chemie AG, 81737 München (DE)
(72) Erfinder: REUTTER-MAIER, Anneliese, 85614 Kirchseeon (DE); BRUNNER, Markus, 81369 München (DE); DASSLER, Tobias, 81825 München (DE)
(74) Vertreter: Potten, Holger
(86) Internationale Anmeldenummer: PCT/EP2012/062236
(87) Internationale Veröffentlichungsnummer: WO 2013/000864

(56) Entgegenhaltungen:
- EP-A1- 1 234 874
- EP-A1- 2 246 420
- WO-A1-2006/088231
- WO-A1-2011/065469

## Beschreibung

Die Erfindung betrifft ein Verfahren zur fermentativen Produktion von natürlichem L-Cystein.

Die Aminosäure L-Cystein wird beispielsweise als Lebensmittelzusatzstoff (insbesondere in der Backmittelindustrie), als Einsatzstoff in der Kosmetik, sowie als Ausgangsprodukt für die Herstellung von Pharmawirkstoffen (insbesondere N-Acetyl-Cystein und S-Carboxy- Methyl-Cystein) verwendet und ist somit von wirtschaftlicher Bedeutung.

L-Cystein nimmt in allen Organismen eine Schlüsselposition im Schwefelmetabolismus ein und wird in der Synthese von Proteinen, Glutathion, Biotin, Liponsäure, Methionin und anderen schwefelhaltigen Metaboliten verwendet. Zudem dient L-Cystein als Vorläufer für die Biosynthese von Coenzym A. Die Biosynthese von L-Cystein wurde in Bakterien, insbesondere in Enterobakterien, detailliert untersucht und ist ausführlich in Kredich (1996, Biosynthesis of cysteine, p. 514-527. In F. C. Neidhardt, R. Curtiss III, J. L. Ingraham, E. C. C. Lin, K. B. Low, B. Magasanik, W. S. Reznikoff, M. Riley, M. Schaechter, and H. E. Umbarger (ed.), Escherichia coli and Salmonella: cellular and molecular biology, 2nd ed. ASM Press, Washington, D.C.) beschrieben.

Neben der klassischen Gewinnung von L-Cystein mittels Extraktion aus keratinhaltigem Material wie Haaren, Borsten, Hörnern, Hufen und Federn oder mittels Biotransformation durch enzymatische Umsetzung von Vorstufen wurde vor einigen Jahren auch ein Verfahren zur fermentativen Herstellung von L-Cystein entwickelt. Der Stand der Technik bezüglich der fermentativen Gewinnung von L-Cystein mit Mikroorganismen ist ausführlich z.B. in US6218168B1, US5972663A, US2004038352A2, CA2386539A2, EP1769080, und EP2138585 beschrieben. Als bakterielle Wirtsorganismen werden dabei u.a. Stämme der Gattung Corynebakterium sowie Vertreter aus der Familie der Enterobacteriaceae, wie z.B. *Escherichia coli* oder *Pantoea ananatis* verwendet.

Neben der klassischen Vorgehensweise, um durch Mutation und Selektion zu verbesserten L-Cystein-Produzenten zu gelangen, wurden auch gezielt genetische Veränderungen an den Stämmen vorgenommen, um eine effektive L-Cystein-Überproduktion zu erreichen.

So führte das Einbringen eines cysE-Allels, das für eine Serin-O-Acetyl-Transferase mit einer verminderten Feedback-Hemmung durch L-Cystein kodiert, zu einer Steigerung der Cystein-Produktion (US6218168B1). Durch ein feedback-resistentes CysE-Enzym wird die Bildung von O-Acetyl-L-Serin, der direkten Vorstufe von L-Cystein, weitgehend vom L-Cystein-Spiegel der Zelle entkoppelt.

O-Acetyl-L-Serin wird aus L-Serin und Acetyl-CoA gebildet. Daher ist die Bereitstellung von L-Serin in ausreichender Menge für die L-Cystein-Produktion von großer Bedeutung. Dies kann durch das Einbringen eines serA-Allels, das für eine 3-Phosphoglycerat-Dehydrogenase mit einer verminderten Feedback-Hemmbarkeit durch L-Serin kodiert, erreicht werden. Dadurch wird die Bildung von 3-Hydroxypyruvat, einer Vorstufe von L-Serin, weitgehend vom L-Serin-Spiegel der Zelle entkoppelt. Beispiele für derartige SerA-Enzyme sind in EP0620853, US7582460B2, und EP0931833 beschrieben.

Darüber hinaus ist bekannt, dass die L-Cystein-Ausbeute in der Fermentation dadurch erhöht werden kann, dass Gene abgeschwächt oder zerstört werden, die für L-Cystein-abbauende Enzyme kodieren, wie z.B. die Tryptophanase TnaA oder die Cystathionine-β-Lyasen MalY oder MetC (EP1571223).

Die Steigerung des Transports von L-Cystein aus der Zelle ist eine weitere Möglichkeit, um die Produkt-Ausbeute im Medium zu erhöhen. Dies kann durch Überexpression sogenannter EffluxGene erreicht werden. Diese Gene kodieren für membrangebundene Proteine, die den Export von L-Cystein aus der Zelle vermitteln. Verschiedene Effluxgene für den L-Cystein-Export wurden beschrieben (US5972663A, US2004038352A2, US2005221453, WO2004113373).

Der Export von L-Cystein aus der Zelle in das Kulturmedium hat folgende Vorteile:
1) L-Cystein wird kontinuierlich aus dem intrazellulären Reaktionsgleichgewicht entzogen mit der Folge, dass der Spiegel dieser Aminosäure in der Zelle niedrig gehalten wird und somit die Feedback-Inhibiton von sensitiven Enzymen durch L-Cystein unterbleibt:

   (1) L-Cystein (intrazellulär) ⇆ L-Cystein (Medium)
2) Das in das Medium ausgeschiedene L-Cystein wird in Gegenwart von Sauerstoff, der während der Kultivierung in das Medium eingebracht wird, zum Disulfid L-Cystin oxidiert (US5972663A):

   (2) 2 L-Cystein + 1/2 O₂ ⇆ L-Cystin + H₂O

   Da die Löslichkeit von L-Cystin in wässriger Lösung bei einem neutralen pH-Wert v.a. im Vergleich zu L-Cystein nur sehr gering ist, fällt das Disulfid schon bei einer niedrigen Konzentration aus und bildet einen weißen Niederschlag:

   (3) L-Cystin (gelöst) ⇆ L-Cystin (Niederschlag)

   Durch die Präzipitation von L-Cystin wird der Spiegel des im Medium gelösten Produkts abgesenkt, wodurch auch jeweils das Reaktionsgleichgewicht von (1) und (2) auf die Produktseite gezogen wird.
3) Der technische Aufwand für die Reinigung des Produkts ist bedeutend geringer, wenn die Aminosäure direkt aus dem Kulturmedium gewonnen werden kann, als wenn das Produkt intrazellulär akkumuliert und zuerst ein Zellaufschluss erfolgen muss.

Bei der Oxidation von zwei Molekülen L-Cystein entsteht das Disulfid L-Cystin. Diese Reaktion ist reversibel, was bedeutet, dass L-Cystin durch Reduktion wieder in L-Cystein überführt werden kann. Wird L-Cystin nach Abtrennung von den Zellen (z.B. mit einem Dekanter) mittels Elektrolyse wieder zu L-Cystein reduziert, so bedingt diese chemische Umsetzung, dass solches L-Cystein nicht als natürlich nach Aromenverordnung deklariert werden darf.

Nach der EU Aromenverordnung (1334/2008 Artikel 22 der Verordnung zur Neuordnung lebensmittelrechtlicher Kennzeichnungsvorschriften) sind natürliche Aromen wie folgt definiert: "Natürlichte" Aromastoffe sind chemisch definierte Stoffe mit Aromaeigenschaften, die natürlich vorkommen und in der Natur nachgewiesen wurden. Sie werden durch geeignete physikalische, enzymatische oder mikrobiologische Verfahren aus pflanzlichen, tierischen oder mikrobiologischen Ausgangsstoffen gewonnen, die als solche verwendet oder mittels eines oder mehrerer herkömmlicher Lebensmittelzubereitungsverfahren (einschließlich mikrobiologischer Prozesse wie Fermentieren, siehe Anhang II) für den menschlichen Verzehr aufbereitet werden.

In diesem Sinne wird der Begriff "natürlich" auch in vorliegender Anmeldung benutzt. Es besteht ein großes Interesse am Einsatz natürlicher Rohstoffe bei der Aromenherstellung. Bisher existierte allerdings kein Verfahren zur fermentativen Herstellung von natürlichem Cystein.

Es gibt eine Reihe von Verbindungen, welche die Oxidation von SH-Gruppen katalysieren können; so ist bekannt, dass Schwermetallsalze wie Eisen- oder Zinksalze essentielle Zusätze bei Fermentationen sind und dass diese Komponenten die Oxidation von Cystein zu Cystin effektiv katalysieren können (US2008190854A2).

In US6218168B1 wird beschrieben, dass das Fermentationsmedium zur Produktion von L-Cystein und seinen Derivaten eine Eisenkonzentration von 15 mg/l (75 mg/l Eisensulfat-Heptahydrat) enthält. EP1389427A1 offenbart ein Medium zur fermentativen Produktion von L-Cystein, L-Cystin und Thiazolidin, in der die Eisenkonzentration mit 14,9 mg/l (74 mg/l Eisensulfat-Heptahydrat) angegeben ist.

In US2010/0093045A1 wird ein Medium zur Produktion von L-Cystein beschrieben, das nur 2 mg/l Eisen (10 mg/l Eisensulfat-Heptahydrat) enthält. Dieses Medium wird allerdings nur für den Einsatz in Schüttelkolben im Labormaßstab (Medienvolumen 20 ml) und nicht für die Fermentation in einem Produktionsfermenter verwendet. EP2133429A1 erwähnt ein Kulturmedium für die Produktion von L-Cystein, L-Cystin, seinen Derivaten oder einer Mischung daraus, das nur 0,34 mg/l Eisen (1,7 mg/l Eisensulfat-Heptahydrat) enthält. Auch dieses Medium ist nicht für einen Einsatz in der Fermentation, sondern für Anzuchten in Röhrchen (Medienvolumen 2 ml) beschrieben. Üblicherweise werden unter diesen Kultivierungsbedingungen (batch-Kultur, schlechte Sauerstoffversorgung, keine pH-Regulierung) nur sehr niedrige Zelldichten (ca. 0,5 bis 2 g/l Biotrockenmasse) und geringe Produktausbeuten erreicht. Dies wird anhand der in Schüttelkolben oder Röhrchen erzielten L-Cystein-Ausbeuten von 0,25 g/l (US2003/0077766A1), 0,3 g/l (EP2133429A1) bzw. 1,2 g/l (US2010/0093045A1) deutlich.

WO 2011/065469 A1 offenbart Verfahren zur fermentativen Herstellung von L-Cystein, bei dem auch L-Cystin sowie L-Cystein-verwandte Substanzen, z.B. Thiazolidin, hergestellt werden. Dabei werden Enterobacteriaceae, z.B. E.coli, die ggf. eine reduzierte feedback-Hemmung durch L-Cystein und/oder L-Serin und/oder eine Überexpression eines Effluxgens aufweisen, in einem Fermentationsmedium mit einer Eisenkonzentration von 0,34 mg/l kultiviert.

EP 2 246 420 A1 beschreibt Verfahren zur fermentativen Herstellung von L-Cystein und verwandten Substanzen in Enterobacteriaceae, z.B. E.coli, durch Überexpression des Membrantunnelproteins TolC. Die Mikroorganismen können weiterhin so verändert sein, dass sie reduzierte Aktivität eines L-Cystein abbauenden Enzyms, reduzierte feedback-Hemmung durch L-Cystein oder erhöhte Expression eines weiteren Effluxgens aufweisen. Die L-Cysteinherstellung findet bei einer Eisenkonzentration von 0,36 mg/l des Produktionsmedium statt.

EP 1 234 874 A1 bezieht sich auf Verfahren zur fermentativen Herstellung von L-Cystein und L-Cystein-Derivaten in coryneformen Bakterien, die ggf. eine reduzierte feedback-Hemmung durch L-Cystein oder eine reduzierte Abbaurate von L-Cystein aufweisen, wobei die Fermentation in einem Medium mit einer Eisenkonzentration von 2 mg/l stattfindet.

WO 2006/088231 A1 offenbart Verfahren zur fermentativen Herstellung von nicht-aromatischen L-Aminosäuren, z.B. L-Cystein, in Enterobacteriaceae, in welchen die Expression des csrA Gens (carbon storage regulator) ausgeschaltet wurde. Das zur Herstellung von Cystein vorgeschlagenen Fermentationsmedium (siehe US6218168) hat eine Eisenkonzentration von 15 mg/l.

In Produktionsfermentern werden jedoch höhe Zelldichten angestrebt, um infolge der biomassespezifischen Produktbildungsraten entsprechend hohe volumetrische Ausbeuten zu erzielen, die erst ein wirtschaftliches Verfahren im industriellen Maßstab ermöglichen. Die Qualität eines industriellen Prozesses zur mikrobiologischen Stoffproduktion wird in der Regel anhand der Produktivität beurteilt. Diese Größe beschreibt die gebildete Gesamtproduktmenge pro Liter Medium pro Fermentationszeit.

Ein Nachteil bei den beschriebenen Verfahren zur fermentativen L-Cystein-Produktion ist, dass die Aminosäure in der Kulturbrühe in verschiedenen Formen vorliegt. Neben dem präzipitierten L-Cystin im Niederschlag findet man im Kulturüberstand lösliches L-Cystein, aber auch L-Cystin in gelöster Form und Thiazolidin (US6218168B1, US5972663A, CA2386539A2). Dieses Thiazolidin (2-Methyl-thiazolidin-2,4-dicarbonsäure) stellt das Kondensationsprodukt aus L-Cystein und Pyruvat dar, das in einer rein chemischen Reaktion gebildet wird.

Unter dem Begriff "Gesamtcystein" werden im Rahmen dieser Erfindung L-Cystein und die daraus abgeleiteten Verbindungen L-Cystin und Thiazolidin, die während der Fermentation gebildet werden und im Kulturüberstand sowie im Niederschlag akkumulieren, zusammengefasst.

Bei den bekannten Verfahren variiert am Ende der Fermentation die Zusammensetzung des Gesamtcysteins: der Anteil des präzipitierten L-Cystins beträgt zwischen 40-66% (US5972663A, CA2386539A2), die restlichen 34-60% liegen im Kulturüberstand in Form von löslichen Produkten - überwiegend als L-Cystein und Thiazolidin - vor. Diese Produktheterogenität erschwert die Gewinnung und Reinigung des Zielprodukts natürliches L-Cystein aus der Kulturbrühe.

Daher wäre ein Verfahren wünschenswert, bei dem als Endprodukt vorwiegend lösliches L-Cystein anfällt. Außerdem sollte möglichst kein Thiazolidin gebildet werden. Die Reinigung des Zielprodukts L-Cystein aus dem Kulturüberstand wäre bei so einem Prozess erheblich einfacher, denn das als Präzipitat vorliegende L-Cystin kann zusammen mit den Zellen durch einen einfachen Separatorschritt abgetrennt werden und das lösliche L-Cystein über Ionentauscheradsorption isoliert werden.

Aufgabe der vorliegenden Erfindung ist es, ein Verfahren zur gezielten Kontrolle der Anteile an L-Cystein, L-Cystin und Thiazolidin am Gesamtcystein in einem Fermentationsmedium bei der fermentativen Herstellung von natürlichem L-Cystein in einem Produktionsfermenter mit einem Nennvolumen ≥ 1 m³ zur Verfügung zu stellen, bei dem ein Mikroorganismenstamm aus der Familie der Enterobacteriaceaein einem Fermentationsmedium kultiviert wird wobei das Endprodukt überwiegend in Form von löslichem L-Cystein im Kulturmedium vorliegt.

Die Aufgabe wird gelöst durch ein Verfahren, das dadurch gekennzeichnet ist, dass die Eisenkonzentration maximal 8 mg/l und minimal 0,6 mg/l beträgt und dadurch erreicht wird, dass dadurch im Fermentationsmedium der L-Cystein-Anteil am Gesammtcystein auf mindestens 65% gesteiget wird.

Die Eisenkonzentration im Fermentationsmedium liegt bevorzugt bei maximal 4 mg/l, besonders bevorzugt bei maximal 2 mg/l Eisen. Es wurde gefunden, dass bei diesen geringen Eisenkonzent rationen ein deutlich homogeneres Produktspektrum in der Fermenterkultur vorliegt, da überwiegend lösliches L-Cystein und präzipitiertes L-Cystin, aber kein Thiazolidin als Produkte gebildet werden.

Ein überraschender Befund war, dass in einem Medium mit diesen geringen Eisenkonzentrationen der L-Cystein-Anteil am Gesamtcystein gesteigert werden kann und am Ende der Fermentation bei mindestens 65 % liegt. Dieser Befund ist besonders deshalb erstaunlich, da Fermentationsmedien, die zur Produktion von Aminosäuren, speziell von L-Cystein und seinen Derivaten, mit Enterobakterien beschrieben sind, mindestens 14,9 mg/l Eisen enthalten (US6218168B1).

Ein weiterer Vorteil der vorliegenden Erfindung ist, dass es im Unterschied zu bekannten Verfahren (EP1389427) bei dem erfindungsgemäßen Verfahren nicht notwendig ist, reduzierende Agenzien wie Vitamin C, Vitamin E oder Ameisensäure und deren Salze, zur Stabilisierung von L-Cystein während des Prozesses zuzugeben.

Die Cystein-Bildungsphase des erfindungsgemäßen Fermentationsverfahrens beginnt mit dem Zeitpunkt, ab dem erstmals L-Cystein in der Kulturbrühe nachgewiesen werden kann und dauert bis zum Ende der Kultivierung an. Typischerweise beginnt diese Phase 2 Stunden nach der Inokulation des Produktionsfermenters.

Im Unterschied zu im Stand der Technik beschriebenen Verfahren (US2003/0077766A1, EP2133429A1, US2010/0093045A1) werden mit dem erfindungsgemäßen Fermentationsverfahren Zelldichten von mindestens 20 g/l, bevorzugt von mindestens 30 g/l, besonders bevorzugt von mindestens 40 g/l Biotrockenmasse und volumetrische Produktausbeuten von mindestens 10 g/l Cystein erzielt.

Als Mikroorganismen für das erfindungsgemäße Verfahren werden Vertreter aus der Familie der Enterobacteriaceae, bevorzugt Vertreter der Gattungen *Escherichia* oder *Pantoea,* ganz besonders bevorzugt Stämme der Spezies *E. coli* oder *P. ananatis* eingesetzt.

Von diesen Mikroorganismenstämmen sind wiederum Stämme bevorzugt, die entweder eine veränderte Serin-O-Acetyl-Transferase besitzen, die im Vergleich zu dem entsprechenden Wildtypenzym eine um mindestens den Faktor 2 verminderte Feedback-Hemmung durch L-Cystein aufweist, oder die durch Überexpression eines Effluxgens einen um mindestens den Faktor 2 erhöhten Cystein-Export aus der Zelle im Vergleich zu einer Wildtypzelle aufweisen. Besonders bevorzugte Mikroorganismenstämme sind solche, die sowohl eine Serin-O-Acetyl-Transferase besitzen, die im Vergleich zu dem entsprechenden Wildtypenzym eine um mindestens den Faktor 2 verminderte Feedback-Hemmung durch L-Cystein aufweist, als auch durch Überexpression eines Effluxgens einen um mindestens den Faktor 2 erhöhten Cystein-Export aus der Zelle im Vergleich zu einer Wildtypzelle aufweisen. Derartige Stämme sind beispielsweise aus US6218168B1 und US5972663A bekannt. Ganz besonders bevorzugte Stämme sind solche, die zusätzlich eine veränderte 3-Phosphoglycerat-Dehydrogenase mit einer im Vergleich zu dem entsprechenden Wildtypenzym um mindestens den Faktor 2 verminderten Feedback-Hemmung durch L-Serin besitzen (US7582460B2) und bei denen mindestens ein L-Cystein-abbauendes Enzym soweit abgeschwächt ist, dass in der Zelle nur noch maximal 50 % dieser EnzymAktivität im Vergleich zu einer Wildtypzelle vorhanden ist. Bevorzugte Varianten der Serin-O-Acetyl-Transferase weisen im Vergleich zu dem entsprechenden Wildtypenzym eine um mindestens den Faktor 5, besonders bevorzugt um mindestens den Faktor 10, ganz besonders bevorzugt um mindestens den Faktor 50 verminderte Feedback-Hemmung durch L-Cystein auf.

Das Effluxgen stammt vorzugsweise aus der Gruppe ydeD (US5972663A), yfiK (US2004038352A2), cydDC (WO2004113373), bcr (US2005221453) und emrAB (US2005221453) von E. coli oder dem entsprechend homologen Gen aus einem anderen Mikroorganismus. Unter einem homologen Gen ist zu verstehen, daß die Sequenz dieses Gens zu mindestens 80 % mit der DNA-Sequenz des entsprechenden E. coli-Gens übereinstimmt.

Die Überexpression eines Effluxgens führt im Vergleich zu einer Wildtypzelle bevorzugt zu einem um mindestens den Faktor 5, besonders bevorzugt um mindestens den Faktor 10, besonders bevorzugt um mindestens den Faktor 20 erhöhten Cystein-Export aus der Zelle.

Bevorzugte Varianten der 3-Phosphoglycerat-Dehydrogenase weisen im Vergleich zu dem entsprechenden Wildtypenzym eine um mindestens den Faktor 5, besonders bevorzugt um mindestens den Faktor 10, ganz besonders bevorzugt um mindestens den Faktor 50 verminderte Feedback-Hemmung durch L-Serin auf.

Das L-Cystein-abbauende Enzym stammt vorzugsweise aus der Gruppe Tryptophanase (TnaA) und Cystathionine-β-Lyase (MalY, MetC).

Besonders bevorzugt sind solche Stämme, in denen mindestens eines dieser Enzyme soweit abgeschwächt ist, dass in der Zelle nur noch maximal 10 % der Enzym-Aktivität im Vergleich zu einem Wildtypstamm vorhanden ist. Ganz besonders bevorzugt sind Stämme, in denen mindestens eines dieser Enzyme völlig inaktiviert ist.

Unter einem Produktionsfermenter ist im Sinn der vorliegenden Erfindung ein Fermenter mit einem Nennvolumen ≥ 1 m³ zu verstehen. Bevorzugt wird ein Fermenter mit einem Nennvolumen ≥ 5 m³, besonders bevorzugt mit einem Nennvolumen ≥ 50 m³ verwendet.

Die Kultivierung der Zellen bei der L-Cystein-Produktion wird unter aeroben Wachstumsbedingungen durchgeführt, d.h. in Gegenwart von Sauerstoff. Der Sauerstoff-Gehalt während der Produktionsphase der L-Cystein-Fermentation sollte bei 30 bis 1% O₂-Sättigung, bevorzugt bei 10 bis 1%, besonders bevorzugt bei 5 bis 1% liegen.

Als Kohlenstoffquelle dienen vorzugsweise Zucker, Zuckeralkohole, organische Säuren oder zuckerhaltige Pflanzenhydrolysate. Besonders bevorzugt werden im erfindungsgemäßen Verfahren als Kohlenstoffquelle Glukose, Fruktose, Laktose, Glycerin oder Gemische, die zwei oder mehr dieser Verbindungen enthalten, eingesetzt.

Bevorzugt wird die Kohlenstoffquelle der Kultur so zudosiert, dass der Gehalt der Kohlenstoffquelle im Fermenter während der Cystein-Produktionsphase 10 g/l nicht übersteigt. Bevorzugt ist eine maximale Konzentration von 2 g/l, besonders bevorzugt von 0,5 g/l, ganz besonders bevorzugt von 0,1 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden. Erfindungsgemäß ist es wichtig, dass für die L-Cystein-Fermentation die Eisenkonzentration des Mediums unter 8 mg/l, bevorzugt unter 4 mg/l, besonders bevorzugt unter 2 mg/l liegt. Eine Eisenkonzentration von 0,2 mg/l sollte nicht unterschritten werden.

Des Weiteren können organische Säuren (z.B. Acetat, Citrat), Aminosäuren (z.B. Isoleucin) und Vitamine (z.B. B1, B6) dem Medium zugesetzt werden.

Als komplexe Nährstoffquellen können z.B. Hefeextrakt, Maisquellwasser, Sojamehl oder Malzextrakt zum Einsatz kommen. Die Inkubationstemperatur für mesophile Mikroorganismen wie z.B. *E. coli* oder *P. ananatis* beträgt vorzugsweise 15 - 45 °C, besonders bevorzugt 30 - 37 °C.

Der pH-Wert des Fermentationsmediums liegt während der Fermentation bevorzugt im pH-Bereich von 5,0 bis 8,5, besonders bevorzugt ist ein pH-Wert von 7,0.

Für die Herstellung von L-Cystein und L-Cystein-Derivaten muss während der Fermentation eine Schwefelquelle zugefüttert werden. Bevorzugt kommen dabei Sulfate oder Thiosulfate zum Einsatz. Für die L-Cystein-Fermentation sollte die Thiosulfat-Konzentration des Mediums unter 5 g/l, bevorzugt unter 3 g/l, besonders bevorzugt unter 1 g/l, ganz besonders bevorzugt unter 0,5 g/l liegen, jedoch 0,1 g/l nicht unterschreiten.

Mikroorganismen, die nach dem beschriebenen Verfahren fermentiert werden, sezernieren in einem Batch- oder Fedbatch-Prozess nach einer Anwachsphase in einem Zeitraum von 2 bis 30 Stunden L-Cystein und davon abgeleitete Verbindungen in hoher Effizienz in das Kulturmedium.

Zur weiteren Aufreinigung des Zielprodukts können folgende Schritte durchgeführt werden:
- Separatorschritt zur Abtrennung der Zellen und des als Präzipitat vorliegenden L-Cystins
- Isolierung des L-Cysteins durch Ionentauscheradsorption
- Fällungskristallisation

Derartige Verfahren sind aus dem Stand der Technik bekannt.

### Beispiel 1: Erzeugung von Cystein-Produktionsstämmen

Es wurden die Wildtyp-Stämme *E*. *coli* W3110 (ATCC 27325) und *P*. *ananatis* (ATCC 11530) jeweils mit dem Plasmid pACYC184/cysEX-GAPDH-ORF306 (offenbart in Beispiel 2 von US5972663A) mittels Elektroporation wie in US5972663A beschrieben transformiert. Das Plasmid pACYC184/cysEX-GAPDH-ORF306 enthält neben dem Replikationsursprung und einem Tetracyclin-Resistenzgen auch noch das cysEX-Allel, das für eine Serin-O-Acetyl-Transferase mit einer verminderten Feedback-Hemmung durch L-Cystein kodiert sowie das Effluxgen ydeD (ORF306), dessen Expression durch den konstitutiven GAPDH-Promotor gesteuert wird.

Die Selektion auf Plasmid-tragende Zellen erfolgte auf LB-Agarplatten, die 15 mg/l Tetracyclin enthielten.

Nach einer erneuten Plasmidisolierung mittels dem QIAprep Spin Plasmid Kit (Qiagen GmbH) und einer Restriktionsanalyse wurden die gewünschten Transformanden, d.h. Zellen, die das Plasmid pACYC184/cysEX-GAPDH-ORF306 aufgenommen haben, isoliert und in der Fermentation eingesetzt, wie dies in Beispiel 2 beschrieben ist.

### Beispiel 2: Kultivierung der Cystein-Produktionsstämme bei unterschiedlicher Versorgung mit Eisensulfat-Heptahydrat

Zum Nachweis der Cystein-Produktion wurden die in Beispiel 1 beschriebenen Mikroorganismen in Fermentern im Fed-Batch-Modus mit kontinuierlicher Glukose- und Thiosulfat-Fütterung kultiviert. Als Produktionsfermenter dienten Bioreaktoren mit einem Nennvolumen von 5 m³. Das Animpfmaterial für die Produktionsfermenter wurde in einer zweistufigen Vorkulturführung bereitgestellt.

### Vorkultur 1 (Schüttelkolben):

Je 400 ml LB-Medium mit 15 mg/l Tetracyclin wurden in zehn Erlenmeyerkolben (2000 ml) mit dem jeweiligen Stamm (*E*. *coli* W3110 pACYC184/cysEX-GAPDH-ORF306 bzw. *P. ananatis* pACYC184/cysEX-GAPDH-ORF306) beimpft und für sieben Stunden auf einem Schüttler (150 rpm, 30°C) inkubiert.

### Vorkultur 2 (Vorfermenter ) :

Anschließend wurde die Vorkultur 1 aus zehn Erlenmeyerkolben vereinigt. 4 1 Vorkultur 1 wurden in eine sterile Impfflasche überführt und vollständig in den Vorfermenter mit einem Nennvolumen von 500 1 gepumpt. Das Fermentationsmedium (200 l) enthält 20 g/l Glukose, 10 g/l Trypton (Difco), 5 g/l Hefeextrakt (Difco), 5 g/l (NN₄)₂SO₄, 1,5 g/l KH₂PO₄, 0,5 g/l NaCl, 0,3 g/l MgSO₄ x 7 H₂O, 0,015 g/l CaCl₂ x 2 H₂O, 0,002 g/l FeSO₄ x 7 H₂O, 1 g/l Na₃Citrat x 2 H₂O, 0,005 g/l Vitamin B1, 1 ml/l Spurenelementlösung (bestehend aus 0,15 g/l Na₂MoO₄ x 2H₂O, 2,5 g/l H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O) und 15 mg/l Tetracyclin.

Der pH-Wert im Vorfermenter wurde vor der Inokulation mit einer 25% NH₄OH-Lösung auf 7,0 eingestellt. Während der Fermentation wurde der pH-Wert durch automatische Korrektur mit 25% NH₄OH auf einem Wert von 7,0 gehalten. Die Kulturen wurden mit 200 rpm gerührt und zu Beginn mit 0,5 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 30 ± 1%, eingestellt. Nach Absinken der O₂-Sättigung unter den Soll-Wert wurde eine Regulationskaskade gestartet, um die O₂-Sättigung wieder an den Soll-Wert heranzuführen. Dabei wurde die Gaszufuhr kontinuierlich auf max. 2 vvm erhöht.

Die Kultivierung wurde bei einer Temperatur von 30°C und einem Druck von 50 kPa für 15 h durchgeführt. Nach dieser Inkubation lag die optische Dichte bei 600 nm (OD₆₀₀) zwischen 18 und 20.

### Hauptkultur (Produktionsfermenter) :

Die Fermentation wurde in Fermentern mit einem Nennvolumen von 5 m³ durchgeführt. Das Fermentationsmedium (2300 1) enthält 15 g/l Glukose, 10 g/l Trypton (Difco), 5 g/l Hefeextrakt (Difco), 5 g/l (NH₄)₂SO₄, 1,5 g/l KH₂PO₄, 0,5 g/l NaCl, 0,3 g/l MgSO₄ x 7 H₂O, 0,015 g/l CaCl₂ x 2 H₂O, 1 g/l Na₃Citrat x 2 H₂O und 1 ml Spurenelementlösung (siehe oben), 0,005 g/l Vitamin B1 und 15 mg/l Tetracyclin. Je nach Versuchsansatz wurde das Medium mit 75 mg/l, 40 mg/l, 20 mg/l, 10 mg/l, 3 mg/l oder 0,5 mg/l FeSO₄ x 7 H₂O supplementiert. Der pH-Wert im Produktionsfermenter wurde vor der Inokulation durch Zupumpen einer 25% NH₄OH-Lösung auf 7,0 eingestellt. Während der Fermentation wurde der pH-Wert durch automatische Korrektur mit 25% NH₄OH auf einem Wert von 7,0 gehalten. Zum Animpfen wurden 200 l der Vorkultur 2 in den Fermenterkessel gepumpt. Das Anfangsvolumen betrug somit etwa 2500 1. Die Kulturen wurden zu Beginn mit 120 rpm gerührt und mit 1,5 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 30 ± 1%, eingestellt. Nach Absinken der O₂-Sättigung unter den Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max. 2 vvm) und anschließend die Rührgeschwindigkeit (auf max. 300 rpm) kontinuierlich gesteigert. Die Fermentation wurde bei einer Temperatur von 30°C und einem Druck von 50-60 kPa durchgeführt. Nach 2 h Fermentationszeit erfolgte die kontinuierliche Zufütterung einer Schwefelquelle. in Form einer sterilen 60 % Natrium-Thiosulfat x 5 H₂0 - Stammlösung. Die Fütterungsrate wurde so eingestellt, dass die Thiosulfatkonzentration im Medium zu keiner Zeit 5 g/l überstieg. Sobald der Glukose-Gehalt im Fermenter von anfänglich 15 g/l auf ca. 2 g/l abgesunken war, erfolgte eine kontinuierliche Zudosierung einer 56% Glukose-Lösung. Die Fütterungsrate wurde so eingestellt, dass die Glukosekonzentration im Fermenter 10 g/l fortan nicht mehr überstieg. Die Glukose-Bestimmung wurde mit einem Glukoseanalysator der Firma YSI (Yellow Springs, Ohio, USA) durchgeführt.

Die Fermentationsdauer betrug 24 Stunden. Danach wurden in allen Fermentern Zelldichten von 40 bis 45 g/l Biotrockenmasse bestimmt.

Am Ende der Fermentation wurden Proben entnommen und der Gehalt von L-Cystein und den davon abgeleiteten Derivaten im Kulturüberstand (L-Cystin und Thiazolidin) und im Niederschlag (L-Cystin) jeweils getrennt voneinander bestimmt (s. Tabellen 1 und 2). Zu diesem Zweck wurde prinzipiell der colorimetrische Ninhydrin-Test von Gaitonde verwendet (Gaitonde, M. K. (1967), Biochem. J. 104, 627-633). Dabei ist zu beachten, dass unter den stark sauren Reaktionsbedingungen des Tests nicht nur freies L-Cystein, sondern auch das im Thiazolidin gebundene L-Cystein mit erfasst und quantifiziert wird. Im Kulturüberstand gelöstes L-Cystin wird im Ninhydrin-Test von Gaitonde nach Reduktion mit Dithiothreitol (DTT) in verdünnter Lösung bei pH 8,0 ebenfalls als L-Cystein nachgewiesen. Das im Niederschlag befindliche L-Cystin musste zuerst in 8% Salzsäure aufgelöst werden, bevor es auf dieselbe Art quantifiziert werden konnte.

Zur differentiellen Quantifizierung der im Kulturüberstand gelösten Komponenten L-Cystein, L-Cystin und Thiazolidin wurden zusätzlich zwei weitere Testmethoden angewandt: Die Quantifizierung von freiem L-Cystein erfolgte durch den von Sang-Han Lee et al. (1995, Biochemical and Biophysical Research Communications 213, 837ff) beschriebenen Test mittels 5,5'-Dithiobis-2-nitrobenzoesäure (DTNB), womit freie SH-Gruppen spezifisch nachgewiesen werden können. Die Differenzierung von L-Cystein und den Derivaten L-Cystin und Thiazolidin erfolgte mittels einer HPLC-Methode, wie in Daßler et al. (2000, Molecular Microbiology 36, 1101ff) beschrieben.

**Tabelle 1: Gehalt von L-Cystein und L-Cystein-Derivaten in der Kulturbrühe von E. coli nach 24 h in Abhängigkeit von der Eisenkonzentration im Medium**

| | | ***E. coli*** | | | | |
|---|---|---|---|---|---|---|
| **FeSO₄ x7H₂O [mg/l]** | **Fe [mg/l]** | **Cystein-Gehalt nach 24 h [g/l]** | | | | **Anteil L-Cystein [%]⁵** |
| | | **Überstand** | | | **Niederschlag⁴** | |
| | | L-Cystein¹ | L-Cystin² | Thiazolidin³ | | |
| **75** | **15** | 8,8 | 1,2 | 1,2 | 11,6 | 38,6 |
| **40** | **8** | 14,6 | 1,2 | 0,2 | 6,4 | 65,2 |
| **20** | **4** | 17,6 | 1,0 | 0 | 5,8 | 72,1 |
| **10** | **2** | 19,6 | 1,0 | 0 | 4,0 | 79,7 |
| **3** | **0,6** | 19,8 | 0,8 | 0 | 4,4 | 79,2 |
| **0,5** | **0,1** | 7,2 | 0,8 | 0 | 9,4 | 41,4 |

| | | | | | | |
|---|---|---|---|---|---|---|
| **¹: L-Cystein im Überstand (DTNB-Test)** **²: gelöstes L-Cystin im Überstand (HPLC)** **³: Thiazolidin im Überstand (HPLC)** **⁴: L-Cystin im Niederschlag** **⁵: Anteil von L-Cystein am Gesamtcystein** | | | | | | |

**Tabelle 2: Gehalt von L-Cystein und L-Cystein-Derivaten in der Kulturbrühe von P. ananatis nach 24 h in Abhängigkeit von der Eisenkonzentration im Medium**

| | | ***P. ananatis*** | | | | |
|---|---|---|---|---|---|---|
| **FeSO₄ x7H₂O [mg/l]** | **Fe [mg/l]** | **Cystein-Gehalt nach 24 h [g/l]** | | | | **Abteil L-Cystein [%] ⁵** |
| | | **Überstand** | | | **Niederschlag⁴** | |
| | | L-Cystein¹ | L-Cystin² | Thiazolidin³ | | |
| **75** | **15** | 5,8 | 1,4 | 1,2 | 7,6 | 36,3 |
| **40** | **8** | 10,4 | 1,2 | 0,2 | 4,8 | 62,7 |
| **20** | **4** | 12,6 | 1,2 | 0 | 4,4 | 69,2 |
| **10** | **2** | 14,6 | 1,0 | 0 | 3,8 | 75,3 |
| **3** | **0,6** | 14,8 | 1,0 | 0 | 4,0 | 74,7 |
| **0,5** | **0,1** | 5,2 | 0, 8 | 0 | 7,6 | 38,2 |

| | | | | | | |
|---|---|---|---|---|---|---|
| **¹: L-Cystein im Überstand (DTNB-Test)** **²: gelöstes L-Cystin im Überstand (HPLC)** **³: Thiazolidin im Überstand (HPLC)** **⁴: L-Cystin im Niederschlag** **⁵: Anteil von L-Cystein am Gesamtcystein** | | | | | | |

## Patentansprüche

1. Verfahren zur gezielten Kontrolle der Anteile an L-Cystein, L-Cystin und Thiazolidin am Gesamtcystein in einem Fermentationsmedium bei der fermentativen Herstellung von natürlichem L-Cystein in einem Produktionsfermenter mit einem Nennvolumen ≥ 1 m³ bei dem ein Mikroorganismenstamm aus der Familie der Enterobacteriaceae in dem Fermentationsmedium kultiviert wird, **dadurch gekennzeichnet, dass** die Eisenkonzentration maximal 8 mg/l und minimal 0,6 mg/l beträgt und dadurch erreicht wird, dass dadurch Im Fermentationsmedium der L-Cystein-Anteil am Gesamtcystein auf mindestens 65% gesteigert wird.

2. Verfahren gemäß Anspruch 1, **dadurch gekennzeichnet, dass** die Eisenkonzentration Im Fermentationsmedium bei maximal 4 mg/l Eisen liegt.

3. Verfahren gemäß Anspruch 1 oder 2, **dadurch gekennzeichnet, dass** keine reduzierenden Agenzien zugegeben werden.

4. Verfahren gemäß einem der Ansprüche 1 bis 3, **dadurch gekennzeichnet, dass** als Mikroorganismenstamm ein Vertreter der Gattungen *Escherichia* oder *Pantoea* eingesetzt wird.

5. Verfahren gemäß Anspruch 1 bis 4, **dadurch gekennzeichnet, dass** der Mikroorganismenstamm sowohl eine veränderte Serin-O-Acetyl-Transferase besitzt, die im Vergleich zu dem entsprechenden Wildtypenzym eine um mindestens einen Faktor 2 verminderte Feedback-Hemmung durch L-Cystein aufweist, als auch durch Überexpression eines Effluxgens einen um mindestens den Faktor 2 erhöhten Cystein-Export aus *der* Zelle im Vergleich zu einer Wildtypzelle aufweist.

6. Verfahren gemäß Anspruch 5, **dadurch gekennzeichnet, dass** der Mlkroorganismenstamm zusätzlich eine veränderte 3-Phosphoglycerat-Dehydrogenase mit einer im Vergleich zu dem entsprechenden Wildtypenzym um mindestens den Faktor 2 verminderten Feedback-Hemmung durch L-Serin besitzt und bei dem mindestens ein L-Cystein-abbauendes Enzym soweit abgeschwächt ist, dass in der Zelle nur noch maximal 50 % dieser EnzymAktivität Im Vergleich zu einer Wildtypzelle vorhanden ist.

7. Verfahren gemäß einem der Ansprüche 1 bis 6, **dadurch gekennzeichnet, dass** die Kultivierung der Zellen in einem Fermenter mit einem Nennvolumen ≥ 5 m³ durchgeführt wird.

8. Verfahren gemäß einem der Ansprüche 1 bis 7, **dadurch gekennzeichnet, dass** Im Fermentationsmedium eine Thiosulfat-Konzentration unter 5 g/l vorliegt, eine Konzentration von 0,1 g/l jedoch nicht unterschritten wird.

## Claims

1. Method for the targeted control of the fractions of L-cysteine, L-cystine and thiazolidine in the total cysteine in a fermentation medium during the preparation by fermentation of natural L-cysteine in a production fermenter with a nominal volume ≥ 1 m³, in which a microorganism strain from the Enterobacteriaceae family is cultured in the fermentation medium, **characterized in that** the iron concentration is at a maximum of 8 mg/l and a minimum of 0.6 mg/l and is achieved by increasing the L-cysteine fraction of the total cysteine to at least 65% in the fermentation medium.

2. Method according to Claim 1, **characterized in that** the iron concentration in the fermentation medium is at a maximum of 4 mg/l iron.

3. Method according to Claim 1 or 2, **characterized in that** no reducing agents are added.

4. Method according to any one of Claims 1 to 3, **characterized in that** the microorganism strain used is a representative of the genera *Escherichia* or *Pantoea.*

5. Method according to Claim 1 to 4, **characterized in that** the microorganism strain both has a modified serine O-acetyl transferase which, compared to the corresponding wild type enzyme, has a feedback inhibition by L-cysteine reduced by at least a factor of 2, and also has, as a result of overexpression of an efflux gene, a cysteine export from the cell that is increased by at least a factor of 2 compared to a wild type cell.

6. Method according to Claim 5, **characterized in that** the microorganism strain additionally has a modified 3-phosphoglycerate dehydrogenase with a feedback inhibition by L-serine reduced by at least a factor of 2 compared to the corresponding wild type enzyme, and in which at least one L-cysteine-degrading enzyme has been attenuated such that only a maximum of 50% of this enzyme activity is still present in the cell compared to a wild type cell.

7. Method according to any one of Claims 1 to 6, **characterized in that** the culturing of the cells is carried out in a fermenter with a nominal volume ≥ 5 m³.

8. Method according to any one of Claims 1 to 7, **characterized in that** in the fermentation medium a thiosulfate concentration below 5 g/l is present, but the concentration does not fall below 0.1 g/l.

## Revendications

1. Procédé pour la régulation ciblée des proportions de L-cystéine, de L-cystine et de thiazolidine par rapport à la cystéine totale dans un milieu de fermentation lors de la préparation par fermentation de L-cystéine naturelle dans un fermenteur de production présentant un volume nominal ≥ 1 m³, dans lequel on cultive une souche de micro-organismes de la famille des Enterobacteriaceae dans le milieu de fermentation, **caractérisé en ce que** la concentration en fer est d'au maximum 8 mg/l et d'au minimum 0,6 mg/l et **en ce qu'**on obtient ainsi que la proportion de L-cystéine par rapport à la cystéine totale dans le milieu de fermentation est augmentée à au moins 65%.

2. Procédé selon la revendication 1, **caractérisé en ce que** la concentration en fer dans le milieu de fermentation se situe à au maximum 4 mg de fer/l.

3. Procédé selon l'une quelconque des revendications 1 ou 2, **caractérisé en ce qu'**on n'ajoute pas d'agents réducteurs.

4. Procédé selon l'une quelconque des revendications 1 à 3, **caractérisé en ce qu'**on utilise, comme souche de micro-organismes, un représentant des espèces Escherichia ou Pantoea.

5. Procédé selon la revendication 1 à 4, **caractérisé en ce que** la souche de micro-organismes contient une sérine-O-acétyl-transférase modifiée, qui présente une inhibition par rétroaction par la L-cystéine diminuée d'au moins un facteur 2 par rapport à l'enzyme de type sauvage correspondante et présente également, par surexpression d'un gène d'efflux, un export de cystéine hors de la cellule augmenté d'au moins un facteur 2 par rapport à une cellule de type sauvage.

6. Procédé selon la revendication 5, **caractérisé en ce que** la souche de micro-organismes présente en outre une 3-phosphoglycérate-déshydrogénase modifiée présentant une inhibition par rétroaction par la L-sérine diminuée d'au moins un facteur 2 par rapport à l'enzyme de type sauvage correspondante et dans laquelle au moins une enzyme dégradant la L-cystéine est affaiblie de manière telle qu'il n'existe dans la cellule plus qu'au maximum 50% de cette activité enzymatique par rapport à une cellule de type sauvage.

7. Procédé selon l'une quelconque des revendications 1 à 6, **caractérisé en ce que** la culture de cellules est réalisée dans un fermenteur présentant un volume nominal ≥ 5 m³.

8. Procédé selon l'une quelconque des revendications 1 à 7, **caractérisé en ce que**, dans le milieu de fermentation, il existe une concentration en thiosulfate inférieure à 5 g/l, mais on ne passe pas sous une concentration de 0,1 g/l.
